# EUROPEAN PATENT SPECIFICATION

(11) **EP 1 933 720 B1**
(45) Date of publication and mention of the grant of the patent: **28.09.2016**
(21) Application number: 06802224.3
(22) Date of filing: 24.08.2006
(51) Int. Cl.: A61B 17/04

(54) **VASCULAR OPENING EDGE EVERSION APPARATUSES**
VASKULÄRE ÖFFNUNGSRANDUMKEHRGERÄTE
APPAREIL D'ÉVERSION DU BORD D'UN ORIFICE VASCULAIRE

(30) Priority: 24.08.2005 US 711279 P; 23.12.2005 US 316775; 23.08.2006 US 508662
(43) Date of publication of application: 25.06.2008
(73) Proprietor: Abbott Vascular Inc., Santa Clara, CA 95054 (US)
(72) Inventor: Sibbitt, Wilmer L., Albuquerque, NM 87106 (US); Sibbitt, Randy R., Helena, MT 59601 (US)
(74) Representative: Boult Wade Tennant
(86) International application number: PCT/US2006/033032
(87) International publication number: WO 2007/025018

(56) References cited:
- US-A- 5 957 938
- US-A- 5 976 161
- US-A1- 2004 225 194
- US-A1- 2004 236 354
- US-A1- 2005 033 359
- US-A1- 2005 075 665
- US-B1- 6 443 963

## Description

### Technical Field

The present invention relates to apparatuses for closing punctures and apertures in human and animal tissue and to apparatuses for inserting such an apparatus into such tissue to perform such closure functions.

### Background Art

During angiography and related procedures, catheters are inserted through an incision or puncture in the skin and underlying tissues to access an artery or vein, typically in the groin, neck, or subclavian areas of a patient. The catheter can be inserted through a puncture in the blood vessel and guided to the desired site to perform interventional procedures such as angiography, angioplasty, plaque removal, and infusion of a therapeutic substance. After the procedure is completed and the catheter is removed from the patient, the access hole must be closed to prevent massive hemorrhage. This is conventionally achieved by applying pressure over the blood vessel manually and then by applying a pressure bandage, compressive weight, or clamp device. With conventional methods, the rate of post-puncture hemorrhage is high, which causes considerable complications. This complication is exacerbated by the concomitant use of anticoagulant medications such as heparin or warfarin and by antiplatelet drugs, which are commonly used to treat vascular disease.

Sutures have been used to close access puncture wounds in blood vessels. US05613974 describes a device and method for applying sutures to a vascular puncture. US2004/0093027A1 describes barbed suture-like material that apposes the puncture site. US 2005/0121042 A1 describes a device and method for applying suture to a vascular puncture. Difficulties with these methods include the large number of steps necessary to deploy the needles, capture the suture, withdraw the suture, tie the knot, and cut the suture. In addition, the hole in the blood vessel is often widened by insertion of the instrument, and the suture remains intravascularly on the endothelial surface, and thus can be a nidus for thrombus or intravascular mural hyperplasia with later spontaneous and catastrophic closure of the vessel.

Extravascular plugs have also been proposed for closure of vascular punctures. US05254105 and US05330445 describe an extravascular plug which is slid down the external surface of the catheter or introducer and is placed into the puncture site in this manner. US05643318 relates to a similar device that has its own vessel locator device. US22022822A1 and US2004/0158287A1 describe an extravascular plug that is delivered with a specialized system. US24215232A1 describes an extravascular plug with an intravascular anchor set with a sheath with a detection port. US2005/0085855A1 describes an extravascular collagen plug, held in place with an intravascular anchor, and a device that locks over a piece of suture. US05906631 describes a plug made of hydrophilic material. US06126675 describes an intravascular anchor and a bioabsorble extravascular plug. US06623509 describes a bioabsorbable plug. US06296657 and US06743195 describe an inflatable balloon that puts pressure on the puncture site. US06569185 describes an injectable vascular plug. US06663655 describes a plug that screws in the puncture tract. US2004/0143290 A1 describes a combination of an intraluminal balloon and injectable sealant. Disadvantages to these methods are related to the high likelihood of thrombosis associated with the intravascular plug or anchor, and the presence of collagen or other bioabsorble materials which cause inflammation, activate the clotting cascade, and increase the likelihood of thrombosis, which, in an arterial system, is catastrophic.

Vascular patches have also been used for repairing blood vessels, but usually only for large areas of damage. US05100422 describes a vascular patch that is sutured to the external surface of the damaged blood vessel. US05100422 describes a vascular patch achieved by instilled adhesives and the device for doing such. These are generally impractical for catheter-based methods. US06248124 and US05507744 describe devices and methods that use electrocautery for sealing vascular punctures. This also requires a complicated device, and perforation and thrombosis are very real possibilities.

Vascular clips or staples delivered through a catheter device have also been proposed. These devices have penetrating members that bring the edges of the tissue together. US06695867 describes a clip or staple that is delivered by a specialized device. US06749622 describes a number of different clips with sharpened barbs or ends that include both intra- and extravascular portions, made of metal with memory characteristics. US05861005 describes an arterial staple that is delivered with a specialized device. US05919207 describes a stapling system based on long hooked wires that appose the surfaces, with a small staple gun to close the lesion. US06022372 describes a similar staple gun. US06296657, US06663655, and US06749621 describe a clip that is external to the vessel, but clips the two sides of the puncture together, and a device for achieving such. US 5782861 and US 5964782 describe clip devices composed of two or more prongs or hooks that, depending on the direction of the prongs, can clip together the puncture site from the intra- or extravascular position, through the use of a collar which forces the prongs together or other mechanisms. These clip devices are composed of thick semi-rigid material, and can be placed only with a specialized instruments, and because of the rigidity have great potential to injure or cut the blood vessel. Disadvantages of these clip devices in general include difficulty in retrieving the device if misplaced, the requirement for excessive manipulation, the thickness of the clip material which tends to cut or shear the blood vessel, the large forces that must be used to curve the staples and fix the clips, the increased possibility of tearing the blood vessel, and the general lack of control of the forces being applied to the blood vessel.

Finally, in US 2005/0033359, which discloses the preamble of claim 1, there is described a closure device for use following various surgical procedures to close access openings through the tissue wall while permitting post operative flow through the tissue conduit. The closure device includes a housing having proximal and distal ends, and defining a longitudinal axis. First and second tissue everting members are mounted adjacent the distal end of the housing and first and second jaw members are mounted adjacent the first and second tissue engaging members. The first and second arterial tissue everting members are dimensioned for at least partial positioning within the access opening in the tissue wall. The everting members are deployable in at least a radial outward direction relative to the longitudinal axis of the housing to engage respective opposed tissue portions on opposed sides of the opening and move the tissue portions to an everted condition thereof.; The first and second jaw members are adapted for relative movement between an open position to facilitate positioning about the tissue portions in the everted condition and a closed position to at least partially draw the tissue portions together to an at least partial approximated condition. Electrodes are associated with the first and second jaw members and arranged to contact the respective tissue portions. The electrodes are adapted to be connected to a radiofrequency energy source whereby energy is transmitted through the electrode to seal the tissue positions between the first and second jaw members to substantially close the opening.

Nonetheless, there remains a need for apparatuses that are suitable for closure of vascular punctures or other openings, and that do not suffer from the drawbacks of conventional approaches.

### Disclosure of Invention

The present invention provides apparatuses that are suitable for closure of vascular punctures or other openings, and that do not suffer from the drawbacks of conventional approaches.

According to the present invention there is provided a tissue eversion device having the features of claim 1.

An apparatus according to the present invention comprises a tool that can be inserted through a puncture wound in a blood vessel and opens within the blood vessel by the extension of multiple members. The members engage and/or penetrate the vessel wall from the intralumenal side of the blood vessel, bringing the vessel wall into apposition by the combination of traction and the geometric shape of the members, A closure device can be inserted over the eversion device, and a closure device (which could be an external suture or external clip) placed on the outside of the blood vessel where it seizes and engages the everted edges of the puncture wound as the members prevent the blood vessel wound edges from moving. Finally, the eversion device may be removed or detached leaving the closure device proximal to the everted wound edges.

There are also described methods of using devices according to the present invention, and methods for bringing wound edges into apposition using devices such as those described herein.

Devices according to the present invention utilize a contractible or expandable material with memory characteristics that allows the members of the device to open and engage spontaneously on a puncture wound of a blood vessel. Members of such devices can have textured gripping surfaces, tissue hooks, or penetrators, to seize the vessel wall and stabilize the device. Such devices can use the spontaneous opening and closing characteristics to seize the edges of the puncture site, and close them, resulting in a complete vascular closure. Such devices can be kept in a contracted or expanded state (high energy state of a memory material) by a delivery sheath and assume its functional, closing form (low energy state of a memory material) when pushed off a delivery sheath.

### Brief Description of Drawings

The invention is explained by using examples and corresponding drawings, which are incorporated into and form part of the specification.
Figure 1(a, b, c, d) is a schematic illustration of a puncture wound eversion-retraction device.
Figure 2(a,b,c) is a schematic illustration of a puncture wound eversion-retraction device.
Figure 3(a,b,c,d) is a schematic illustration of a puncture wound eversion-retraction device.
Figure 4(a,b,c,d,e,f,g) is a schematic illustration of a puncture wound eversion-retraction device.
Figure 5(a,b) is a schematic illustration of a puncture wound eversion-retraction device.
Figure 6(a,b) is a schematic illustration of a puncture wound eversion-retraction device.
Figure 7(a,b) is a schematic illustration of a puncture wound eversion-retraction device.
Figure 8(a,b,c,d) is a schematic illustration of a puncture wound eversion-retraction device.
Figure 9(a,b) is a schematic illustration of a puncture wound eversion-retraction device according to the present invention.

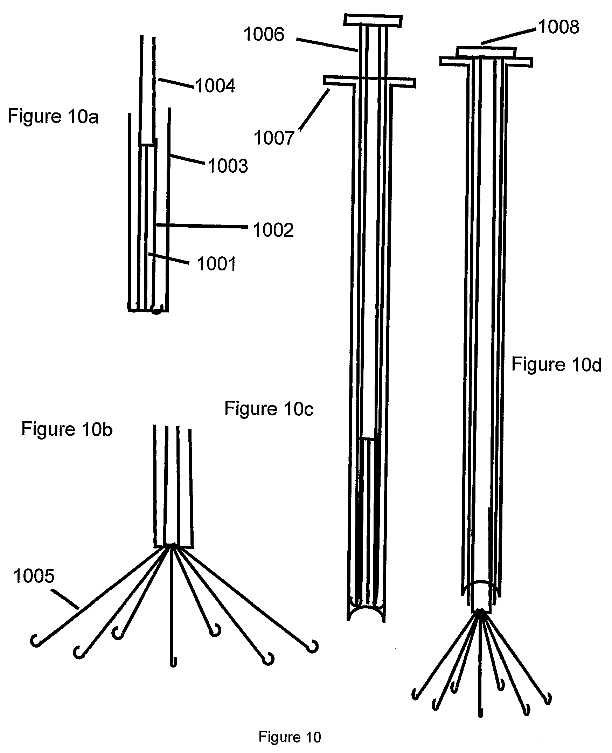

Further examples of a puncture wound eversion-retraction device are shown in Figures 10 to 14.

### Modes for Carrying Out the Invention, and Industrial Applicability

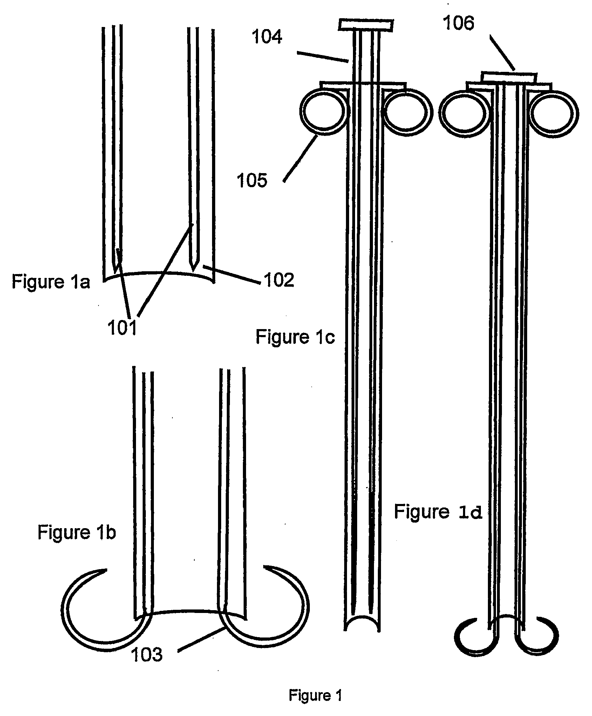

The present invention provides apparatuses for closing a vascular puncture wound or any tissue aperture, for example those resulting from the insertion of a vascular catheter or surgical instrument, trauma or disease. Devices according to the present invention can be inserted in a vascular sheath, the sheath removed or pulled back, and a closure device placed over the everter device. The everter device is activated by extending graspers within the blood vessel. The graspers are pulled up against and penetrate the vascular vessel wall. The wound edges are everted, apposed, and brought up into the closure device. Finally the everted wound edges are closed distal to the graspers by the means of an extravascular clip, extravascular suture, extravascular glue or patch, extravascular heat coagulation, or by staples or sutures that are placed through the lips of the everted wound edges. This behavior can be provided by forming at least a portion of the grasping device of a memory metal or material. The stress free state corresponds to the state at which the apparatus has closed upon the everted edges of a puncture wound of a blood vessel, and the stressed state is when the device is open and seated on the delivery sheath. Examples of tissue closure apposition devices are shown in Figures 1, 2, 3, and 4. The descriptions refer to "vessels" for convenience; however the present invention is applicable to facilitate closure of various types of tissue openings.

Figure 1(a,b,c,d) is a schematic illustration of a puncture wound everter device. Figure 1a is a lateral cutaway view of a puncture wound everter device in the closed state (high energy state). A plurality of grasping members 101 (generally a minimum of two members), for grasping tissue at at least two locations are shown in the retracted state. A sheath 102 contains the grasping members. This sheath can accommodate a guidewire, or in another arrangement can be inserted through a sheath or a closure device and used like a guidewire. Figure 1b is a lateral view of the same device in the extended or opened state (low energy state), where the grasping members 103 are extended, and curl up and engage and/or pierce the blood vessel wall. Figure 1c is a lateral view of the everting device in the retracted state with a plunger mechanism 104 to extend the grasping members and finger flanges or rests 105 to control the device. Figure 1d is the same device with the grasping members extended by pushing a plunger mechanism 106.

In Figure 1 the everter device can be placed into the puncture wound by means of a guidewire that can be accommodated within the sheath 102 in which case the everter device is placed over the guidewire and pushed through the puncture wound into the blood vessel. The closure device is then placed over the everter device and the terminal end brought up against the exterior blood vessel wall. The everter members are then extended and the device pulled up against the blood vessel wall, and the everted wound edges retracted within and held within the closure device. After this the guidewire is removed and the closure mechanism applied by the closure device causing the wound edges to be closed distally to the everting members. At this point the everter members are retracted and both the closure device and everter device removed, leaving the closure mechanism in place on the external surface of the blood vessel.

Alternatively, in Figure 1 the everter device can be placed into the puncture wound by means of a sheath that can accomodate the everter device internally. The everter device sheath 102 is placed within the operating sheath and pushed through the existing sheath into the puncture wound and into the internal lumen of the blood vessel. The operator sheath is then removed and the closure device is then placed over the everter device with the everter device sheath being used as a guidewire. The terminal end of the closure device is brought up against the exterior blood vessel wall. The everter members of the everter device are then extended and the device pulled up against the blood vessel wall, so that the everter members penetrate and seize the blood vessel wall. The everter device is then partially retracted, so that the everted wound edges are retracted within and held within the closure device. The closure mechanism is then applied by the closure device causing the everted wound edges to be closed distally to the everting grasping members. At this point the everter members are retracted and both the closure device and everter device removed, leaving the closure mechanism in place on the external surface of the blood vessel.

Figure 1 presents for illustration purposes 2 active members; the device can comprise as few as two active members (or one, if it grasps the tissue at multiple locations), but can include any plurality, and as many as are practical within applicable design considerations. The tissue engagement features, shown in the figure as sharp hook-like portions of the active members can comprise textured portions or attachments, mating portions with apposing feet, penetrating devices, hooks, teeth, or other adaptations to allow firm grip of the tissue. The members can comprise memory materials to fit within the delivery sheath, to assume a lower profile when delivered, and to expand and engage the vessel wall when extended.

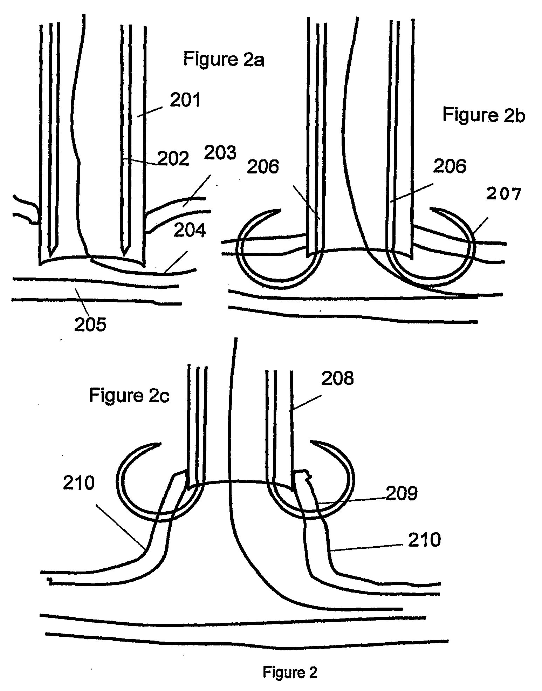

Figure 2 (a,b,c) demonstrates an example of how a puncture wound everter device can be used. Figure 2a is a lateral cutaway view of the everter device after insertion into the blood vessel where 201 is the everter device sheath, 202 is the retracted (high energy state) grasping member, 203 is the proximal vessel wall; 204 is the guidewire, and 205 is the distal vessel wall. Figure 2b demonstrates the everter device with the grasping members extended where 206 is the extended grasping member, and 207 is the grasping member penetrating the proximal vessel wall. Figure 2c shows the everter device with traction, where 208 is the everter device pulled proximally towards the operator, which enlongates, everts, apposes and holds the vessel wall 209, so that a closure device can be placed on the neck of the everted wound margins 210.

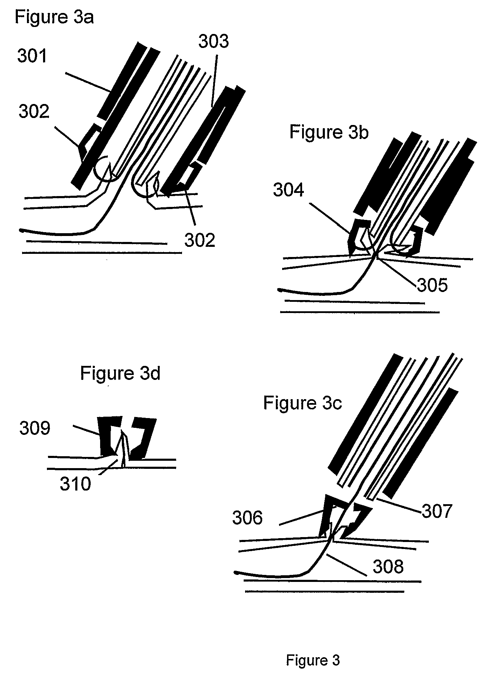

Figure 3 (a,b,c,d) demonstrates how an everter device can be used to apply an extravascular clip or stable. Figure 3a is a lateral cutaway view, where 301 is expulsion portion of the closure devices, 302 is the extravascular clip or staple, and 303 is the extravascular clip or staple delivery sheath. Figure 3b is a view of the clip 304 being delivered onto the neck of the everted wound edges, closing the wound 305. Figure 3c demonstrates the everter device 307 with the members retracted, leaving the clip or staple closed over the guidewire 308. Figure 3d demonstrates the clip or staple left in situ, with the clip 309 left in place, completely closing the puncture wound edges 310.

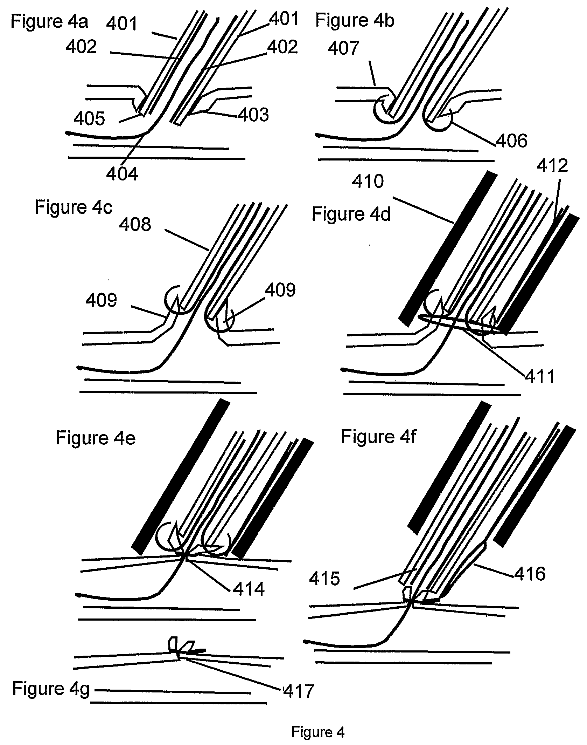

Figure 4 (a,b,c,d,e,f,g) is a demonstration of a method to insert a closure device, in this case an extravascular suture. Figure 4a is a lateral view where the gripper sheath 401 is placed over the guidewire 404, so that the distal edge of the sheath has been pushed internally relative to the proximal blood vessel wall 403. The gripper members 402 have not been extended. In Figure 4b the gripper tines 406 are extended penetrating or gripping the proximal blood vessel wall 407. In Figure 4c traction is then placed on the gripper sheath and members 408, which pull and evert the wound edges 409. In Figure 4d the suture introducer sheath 410 is placed over the gripper sheath, and the suture 411 is tightened by pulling on the suture drawstring contained in 412. In Figure 4e the suture is completely closed around the guidewire 414, closing the puncture. In Figure 4f the gripper members 415 are retracted within the everter gripper sheath, and the suture loop 416 is cut. In Figure 4g, if there is no bleeding, the guidewire is removed leaving at 417 an external suture closure of the blood vessel puncture wound.

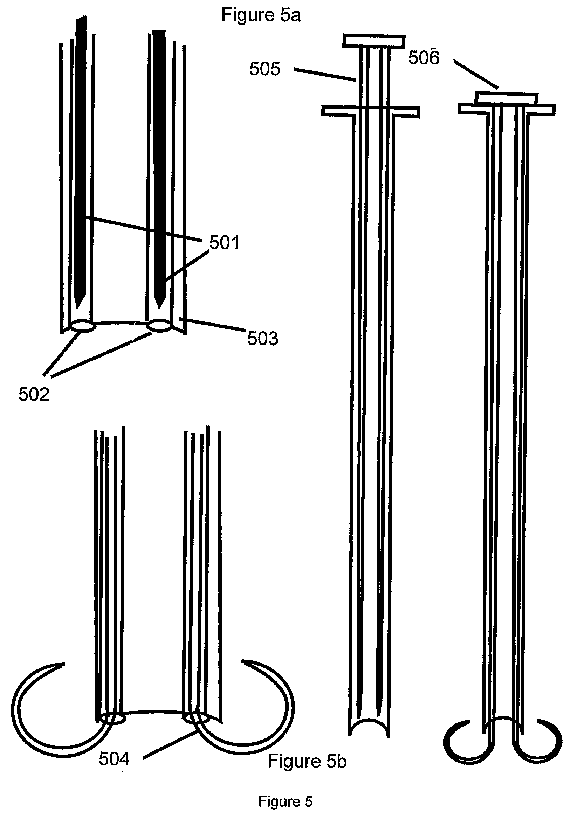

Figure 5(a,b,) is another schematic illustration of a puncture wound everter device, similar to that of Figure 1. Figure 5a is a lateral cutaway view of a puncture wound everter device in the closed state (high energy state). 501 are the plurality of grasping members (comprising a minimum of two members) in the retracted state; these are constrained within internal lumens 502, which are held within the everter device sheath. 503 is the sheath that contains the grasping members. This sheath can accommodate a guidewire, or in another arrangement can be inserted through a sheath or a closure device and used like a guidewire. Figure 5b is a lateral view of the same device in the extended or opened state (low energy state), where the grasping members 504 are extended, and curl up and engage and/or pierce the blood vessel wall. This device would otherwise be inserted, operated, and used identically to the example in Figure 1. Figure 5 presents for illustration purposes two active members; the device can comprise as few as two active members, but can include any plurality, and as many as are practical within applicable design considerations. The tissue engagement features, shown in the figure as sharp hook-like portions of the active members can comprise textured portions or attachments, mating portions with apposing feet, penetrating devices, hooks, teeth, or other adaptations to allow firm grip of the tissue. The members can comprise memory materials to fit within the delivery sheath, to assume a lower profile when delivered, and to expand and engage the vessel wall when extended.

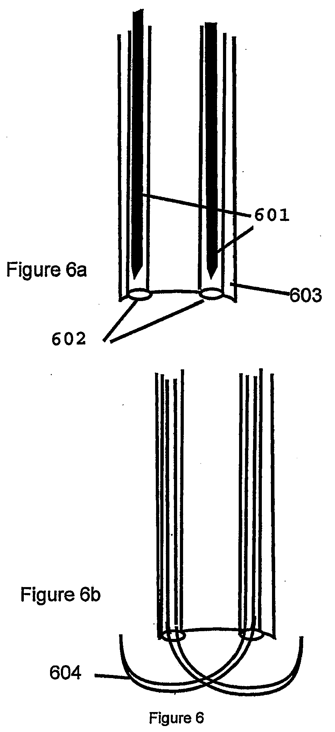

Figure 6(a,b) is another schematic illustration of a puncture wound everter device, similar to that of Figure 1. Figure 6a is a lateral cutaway view of a puncture wound everter device in the closed state (high energy state). 601 are the plurality of grasping members (comprising a minimum of two members) in the retracted state; these are constrained within internal lumens 602 (or not), which are held within the everter device sheath 603 i.e., the sheath that contains the grasping members. In this case the members are extended in a cross-wise function across the sheath, the purpose being that the wound edges are more efficiently and mechanically brought into apposition by the method of retraction. The sheath can accommodate a guidewire, or in another arrangement can be inserted through a sheath or a closure device and used like a guidewire. Figure 6b is a lateral view of the same device in the extended or opened state (low energy state), where the grasping members 604 are extended, and curl up and engage and/or pierce the blood vessel wall. This device would otherwise be inserted, operated, and used identically to the example in Figure 1. Figure 6 presents for illustration purposes two active members; the device can comprise as few as two active members, but can include any plurality, and as many as are practical within applicable design considerations. The tissue engagement features, shown in the figure as sharp hook-like portions of the active members can comprise textured portions or attachments, mating portions with apposing feet, penetrating devices, hooks, teeth, or other adaptations to allow firm grip of the tissue. The members can comprise memory materials to fit within the delivery sheath, to assume a lower profile when delivered, and to expand and engage the vessel wall when extended.

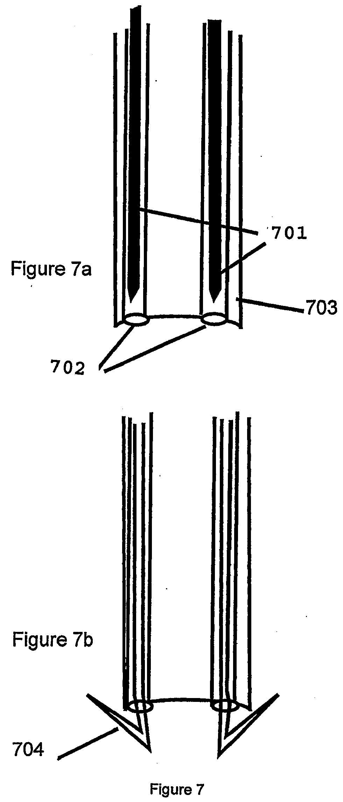

Figure 7(a,b,) is another schematic illustration of a puncture wound everter device, similar to that of Figure 1. Figure 7a is a lateral cutaway view of a puncture wound everter device in the closed state (high energy state). 701 are the plurality of grasping members (comprising a minimum of two members) in the retracted state; these are constrained within internal lumens 702 (or not), which are held within the everter device sheath 703 i.e., the sheath that contains the grasping members. In this case the members comprise at a sharp geometric angle. The sheath can accommodate a guidewire, or in another arrangement can be inserted through a sheath or a closure device and used like a guidewire. Figure 7b is a lateral view of the same device in the extended or opened state (low energy state), where the grasping members 704 are extended, and curl up and engage and/or pierce the blood vessel wall. This device would otherwise be inserted, operated, and used identically to the example in Figure 1. Figure 7 presents for illustration purposes two active members; the device can comprise as few as two active members, but can include any plurality, and as many as are practical within applicable design considerations. The tissue engagement features, shown in the figure as sharp hook-like portions of the active members, can comprise textured portions or attachments, mating portions with apposing feet, penetrating devices, hooks, teeth, or other adaptations to allow firm grip of the tissue. The members can comprise memory materials to fit within the delivery sheath, to assume a lower profile when delivered, and to expand and engage the vessel wall when extended.

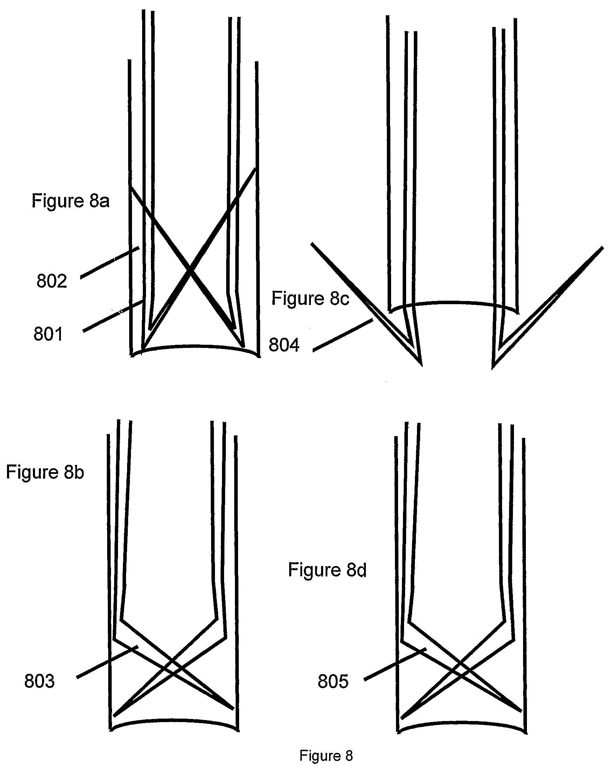

Figure 8(a,b,c,d) is a schematic illustration of a puncture wound everter device. Figure 8a is a lateral cutaway view of a puncture wound everter device in the closed state (high energy state). 801 are the plurality of grasping members (comprising a minimum of two members) in the retracted state; in this case, the members are flexed back within the sheath; and 802 is the sheath that contains the grasping members. This sheath can accommodate a guidewire, or in another arrangement can be inserted through a sheath or a closure device and used like a guidewire. Figure 8b is a lateral view of the same device in the retracted or closed states state (low energy state), where the grasping members 803 are flexed forward. Figure 8c is a lateral view of the same device in the extended or opened state (low energy state), where the grasping members 804 are extended, and flex up and engage and/or pierce the blood vessel wall. This represents the extended form of the devices shown in Figure 8a and Figure 8b. Figure 8d is a lateral view of the same device in the retracted or closed state (high energy state), where the grasping members 805 are retracted, and flexed down. This represents the retracted form of the devices shown in Figure 8a and Figure 8b.

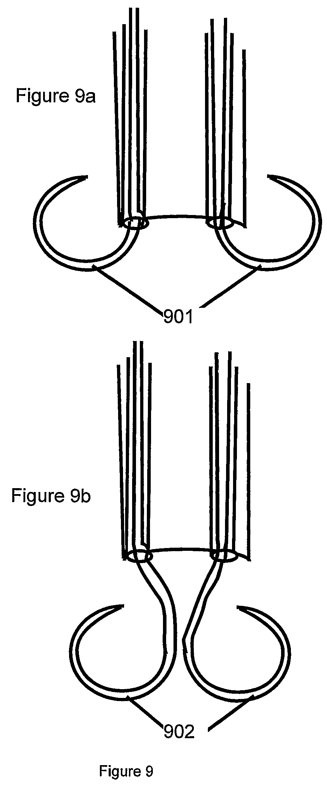

Figure 9(a,b) is a schematic illustration of a puncture wound everter device according to the invention, similar to that of Figure 1. Figure 9a is a lateral cutaway view of a puncture wound everter device in the partially opened state (half-way to low energy state). 901 are the plurality of grasping members (comprising a minimum of two members) in the partially extended state. Figure 9b is a lateral view of the same device in the fully extended or opened state (low energy state), where the grasping members 902 are extended, and curl towards each other after they have engaged and/or pierced the blood vessel wall. In this case the members when fully extended move towards the midline of the sheath, the purpose being that the wound edges are more efficiently and mechanically brought into apposition during extension and retraction. This device would otherwise be inserted, operated, and used identically to the devices in the prior examples. Figure 9 presents for illustration purposes two active members; the device can comprise as few as two active members, but can include any plurality, and as many as are practical within applicable design considerations. The tissue engagement features, shown in the figure as sharp hook-like portions of the active members can comprise textured portions or attachments, mating portions with apposing feet, penetrating devices, hooks, teeth, or other adaptations to allow firm grip of the tissue. The members comprise memory materials to fit within the delivery sheath, to assume a lower profile when delivered, and to expand and engage the vessel wall when extended.

Figure 10 (a,b,c,d) is a schematic illustration of another puncture wound everter device. Figure 10a is a lateral cutaway view of a puncture wound everter device in the closed state (high energy state). 1001 are the plurality of grasping members (comprising a minimum of two members) in the retracted state; in this case, the members are closed within an internal lumen 1002 and/or within a sheath 1003. The grasping members are attached to a solid member 1004 which can be a solid or hollow columnar device with an internal lumen for a guidewire, or can itself be a wire. This sheath can accommodate a guidewire, or in another arrangement can be inserted through a sheath or a closure device and used like a guidewire. Figure 10b is a lateral view of the same device in the extended or open state (low energy state), where the grasping members 1005 are extended into their grasping or extended position. Figure 10c is a lateral view of the everting device in the retracted state with a plunger mechanism 1006 to extend the grasping members and finger flanges or rests 1007 to control the device. Figure 10d is the same device with the grasping members extended by pushing the plunger mechanism 1008.

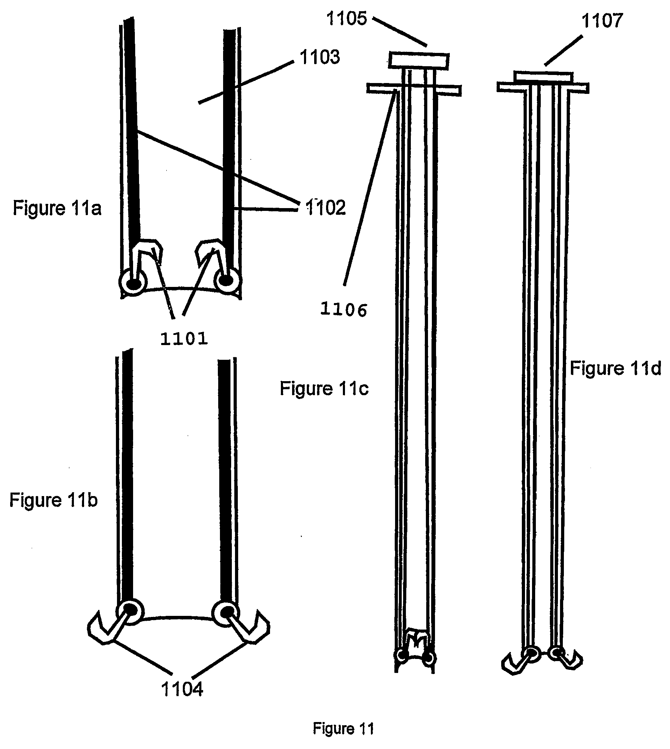

Figure 11 (a,b,c,d) is a schematic illustration of another puncture wound everter device. Figure 11a is a lateral cutaway view of a puncture wound everter device in the closed state (high energy state). 1101 are a plurality of grasping members (comprising a minimum of two members) in the retracted state. In this case, the members can rotate from internally to externally each upon an axis moved by a mechanical mechanism 1102 (which could be a flywheel mechanism or gear and groove axel mechanism) all of which is enclosed within an internal lumen 1103 and/or sheath 1103. Figure 11 b is a lateral view of the same device in the extended or open state, where the grasping members 1104 are extended into their grasping or extended position. Figure 11c is a lateral view of the everting device in the retracted state with a plunger mechanism 1105 to extend the grasping members, and finger flanges or rests 1106 to control the device. Figure 11d is the same device with the grasping members extended by pushing the plunger mechanism 1107.

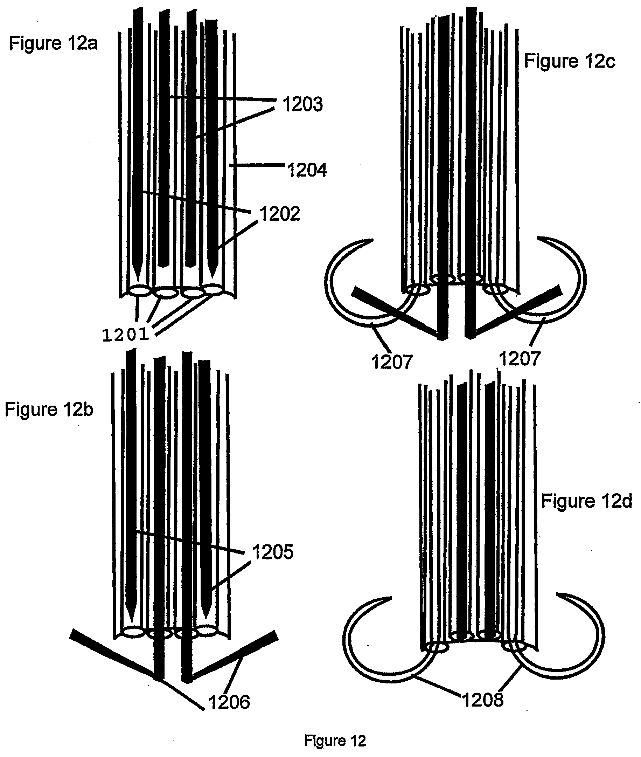

Figure 12 (a,b,c,d) is a schematic illustration of another puncture wound everter device. Figure 12a is a lateral cutaway view of a puncture wound everter device in the closed state (high energy state) that is placed through a puncture wound within a blood vessel. 1201 are internal lumina for the plurality of grasping members 1202 (comprising a minimum of two members) in the retracted state. At least two parallel locator members 1203 are also present in their lumina within an everter device sheath 1204. Figure 12b demonstrates that the grasping members 1205 have not been extended, while the locator members 1206 have been extended. As can be seen, these do not have penetrating surfaces. The locator members are pulled up against the internal edges of the puncture wound, locating the wound margins. In Figure 12c, once the wound margins have been located the grasping members are extended 1207, and these penetrate and grasp the proximal vessel wall. In Figure 12d once the vessel is grasped by the grasping members 1208, the locator members are retracted, and the device is then used identically to the other examples.

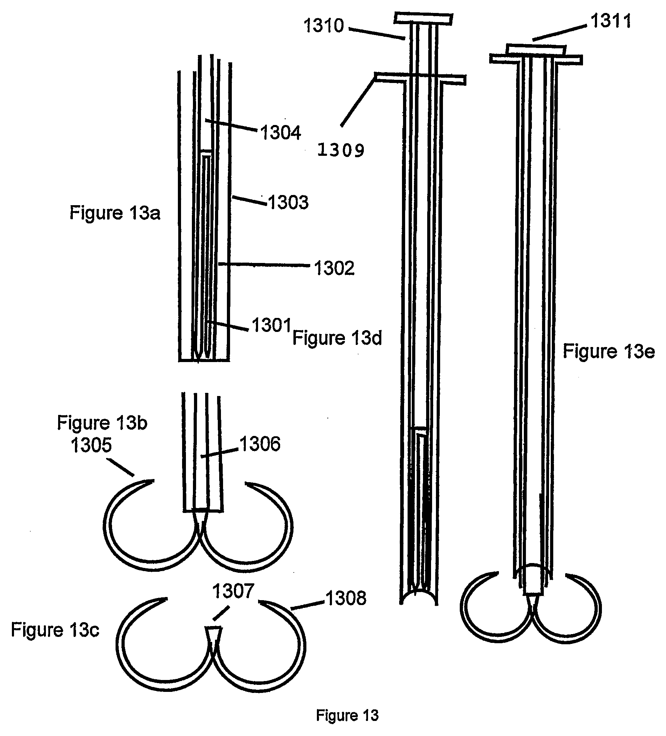

Figure 13 (a,b,c,d,e) is a schematic illustration of another puncture wound everter device. Figure 13a is a lateral cutaway view of a puncture wound everter device in the closed state (high energy state) that is placed through a puncture wound within a blood vessel. 1301 shows the plurality of grasping members (comprising a minimum of two members) in the retracted state within an internal lumen 1302 within the everter device sheath 1303. The grasping members 1301 are attached to an internal columnar structure 1304. Figure 13b illustrates the grasping members 1305 extended, by action of the internal columnar structure 1306 which has been extended. Figure 13c illustrates the grasping members 1308 detached from the columnar structure at a mating area 1307 and expelled independently. Figure 13d is a lateral view of the everting device in the retracted state with a plunger mechanism 1310 to extend the grasping members and finger flanges or rests 1309 to control the device. Figure 13d is the same device with the grasping members extended by pushing the plunger mechanism 1311.

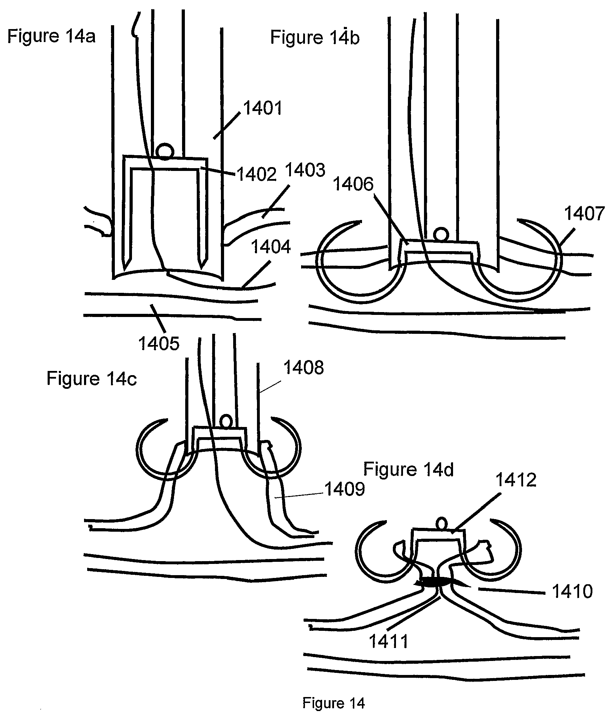

Figure 14 (a,b,c,d) is a view of another example of a gripper device in which a residing memory gripper is used. In Figure 14a the gripper device sheath 1401 with the residing gripper 1402 and a columnar connecter is introduced into the blood vessel 1403 over the guidewire 1404. In Figure 14b after introduction, the gripper 1406 is expelled from the sheath, and the gripping members 1407 then penetrate and grip the proximal tissue surrounding the puncture wound. In Figure 14c traction (pulling) is placed on the gripper sheath 1408 and gripper members on the columnar base, everting the wound edges 1409, In Figure 14d a closure device 1410 is placed over the gripper device and closed on the neck of the everted wound edges, closing the wound edges 1411. The gripping device 1412 is detached from the columnar holder and is held in place and prevented from migrating internally by the closure device 1410 and the closure device is prevented from migrating externally by the gripping device 1412. Thus, this is a combination of both a clip and a suture.

Any part of an apparatus according to the present invention can be made from any of a number of suitable materials, or combinations thereof. In some applications, it can be desirable for members to be of radioopaque materials or be coated to be made radioopaque. Members can be made from bioabsorbable polymers or compounds, non-absorbable alloys and compounds including stainless steel, MP35, Nitinol, Nickel-Titanium alloy, Kevlar, nylon polyester acrylic, gold, platinum, tantalum, niobium, molybdenum, rhodium, palladium silver, hafnium, tungsten, iridium. Materials with memory are useful, as the memory property can provide force for activation of the active members from the open to the closed state. Members can be made in the form of wires, fibers, filaments, small beams, and other extruded, woven, or formed shapes. Examples of suitable materials include piano wire, super elastic memory wire, chromium alloys, alloys of titanium and nickel, and other elastic memory materials. A suitable fabric or coating can be made from a number of suitable materials. In some applications it can be desirable to use flexible polymeric materials with elastomeric properties including polyurethane, polyethylene, polyestenurethane, polyimide, polyethreimide, polycarbonate, polysiloxane, polyvinyls, hydroxyethylmethacrylate, related polymers, copolymers of these or other polymers, or drug-embedded or drug-eluting polymers to prevent coagulation or intimal hyperplasia (such as Taxol), which can be made radiopaque by markers to addition of appropriate radiopaque materials.

The particular sizes and equipment discussed above are cited merely to illustrate particular embodiments of the invention. It is contemplated that the use of the invention may involve components having different sizes and characteristics. It is intended that the scope of the invention be defined by the claims appended hereto.

## Claims

1. A tissue eversion device comprising:
a delivery sheath; and
a tissue engaging element configured to evert the edges of an aperture in a tissue wall, said tissue engaging element comprising a plurality of elongated members (901, 902), each comprising a memory material and having an end adapted to engage tissue, said tissue engaging element having a first configuration in which said elongated members reside within said delivery sheath substantially parallel to an axis of said delivery sheath, and a second configuration in which the elongated members extend beyond the outer surface of the delivery sheath, **characterized in that** when fully extended in the second configuration the elongated members move towards a midline of the delivery sheath before then curling up to engage the tissue wall and then back towards each other.

2. A tissue eversion device as in claim 1, wherein said elongated members have sharpened ends.

3. A tissue eversion device as in claim 1, wherein the tissue engaging element comprises metal.

4. A tissue eversion device as in claim 1, wherein said tissue engaging element comprises bioabsorbable polymer, bioabsorbable compound, non-absorbable alloy, non-absorbable compound, stainless steel, MP35, Nitinol, Nickel-Titanium alloy , Kevlar, nylon polyester acrylic, gold, platinum, tantalum, niobium, molybdenum, rhodium, palladium, silver, hafnium, tungsten, iridium, artificial muscle, shape memory alloy, or a combination thereof.

5. A tissue eversion device as in claim 1, wherein said tissue engaging element is moveable relative to said delivery sheath along an axis of said delivery sheath, and wherein motion of said tissue engaging element along the axis past the end of said delivery sheath moves said tissue engaging element to the second configuration and wherein motion of said tissue engaging element into the end of said delivery sheath moves said tissue engaging element to the first configuration.

6. A tissue eversion device as in claim 1, wherein said tissue engaging element can be actuated to attain the second configuration by action of a plunger and flanges mounted with the device.

7. A tissue eversion device as claim 1, further comprising a plurality of individual lumens within said delivery sheath, and wherein said elongated members are movably mounted within said individual lumens.

## Patentansprüche

1. Gewebeeversionsvorrichtung, die aufweist:
eine Zufuhrhülle; und
ein Gewebegreifelement, das ausgebildet ist, die Kanten einer Öffnung in einer Gewebewand umzustülpen, wobei das Gewebegreifelement mehrere längliche Elemente (901, 902) aufweist, die jeweils ein Gedächtnismaterial umfassen und ein Ende aufweisen, das zum Greifen von Gewebe ausgebildet ist, wobei das Gewebegreifelement eine erste Konfiguration, bei der die länglichen Elemente innerhalb der Zufuhrhülle im Wesentlichen parallel zu einer Achse der Zufuhrhülle aufgenommen sind, und eine zweite Konfiguration aufweist, bei der sich die länglichen Elemente über die äußere Oberfläche der Zufuhrhülle hinaus erstrecken, **dadurch gekennzeichnet, dass** sich die länglichen Elemente, wenn sie vollständig in die zweite Konfiguration ausgefahren sind, in Richtung einer Mittellinie der Zufuhrhülle bewegen, bevor sie sich anschließend einrollen, um die Gewebewand zu greifen, und sich anschließend zurück aufeinander zu bewegen.

2. Gewebeeversionsvorrichtung nach Anspruch 1, wobei die länglichen Elemente angespitzte Enden aufweisen.

3. Gewebeeversionsvorrichtung nach Anspruch 1, wobei das Gewebegreifelement Metall aufweist.

4. Gewebeeversionsvorrichtung nach Anspruch 1, wobei das Gewebegreifelement ein bioabsorbierbares Polymer, eine bioabsorbierbare Verbindung, eine nicht-absorbierbare Legierung, eine nichtabsorbierbare Verbindung, Edelstahl, MP35, Nitinol, eine Nickel-Titan-Legierung, Kevlar, Nylon-Polyester-Acryl, Gold, Platin, Tantal, Niob, Molybdän, Rhodium, Palladium, Silber, Hafnium, Wolf ram, Iridium, einen künstlichen Muskel, eine Formgedächtnislegierung oder eine Kombination hiervon umfasst.

5. Gewebeeversionsvorrichtung nach Anspruch 1, wobei das Gewebegreifelement entlang einer Achse der Zufuhrhülle relativ zur Zufuhrhülle bewegt werden kann und wobei die Bewegung des Gewebegreifelements entlang der Achse über das Ende der Zufuhrhülle hinaus das Gewebegreifelement in die zweite Konfiguration überführt und wobei die Bewegung des Gewebegreifelements in das Ende der Zufuhrhülle das Gewebegreifelement in die erste Konfiguration überführt.

6. Gewebeeversionsvorrichtung nach Anspruch 1, wobei das Gewebegreifelement durch einen Kolben und Flansche, die an der Vorrichtung angebracht sind, so betätigt werden kann, dass es die zweite Konfiguration einnimmt.

7. Gewebeeversionsvorrichtung nach Anspruch 1, die mehrere individuelle Lumen innerhalb der Zufuhrhülle aufweist, wobei die länglichen Elemente innerhalb der individuellen Lumen beweglich angebracht sind.

## Revendications

1. Dispositif d'éversion de tissu comprenant :
une gaine de distribution ; et
un élément pénétrant le tissu configuré pour retourner les bords d'une ouverture dans une paroi de tissu, ledit élément pénétrant le tissu comprenant une pluralité d'éléments allongés (901, 902), comprenant chacun un matériau à mémoire et ayant une extrémité adaptée pour pénétrer le tissu, ledit élément pénétrant le tissu ayant une première configuration dans laquelle lesdits éléments allongés résident à l'intérieur de ladite gaine de distribution sensiblement parallèles à un axe de ladite gaine de distribution et une deuxième configuration dans laquelle les éléments allongés s'étendent au-delà de la surface extérieure de la gaine de distribution, **caractérisé en ce que**, lorsqu'ils sont entièrement étendus dans la deuxième configuration, les éléments allongés se déplacent d'abord vers une ligne médiane de la gaine de distribution puis se courbent pour pénétrer la paroi de tissu et enfin reviennent l'un vers l'autre.

2. Dispositif d'éversion de tissu selon la revendication 1, dans lequel lesdits éléments allongés ont des extrémités aiguisées.

3. Dispositif d'éversion de tissu selon la revendication 1, dans lequel l'élément pénétrant le tissu comprend un métal.

4. Dispositif d'éversion de tissu selon la revendication 1, dans lequel ledit élément pénétrant le tissu comprend un polymère bioabsorbable, un composé bioabsorbable, un alliage non absorbable, un composé non absorbable, un acier inoxydable, du MP35, du Nitinol, un alliage nickel-titane, du kevlar, du nylon, du polyester acrylique, de l'or, du platine, du tantale, du niobium, du molybdène, du rhodium, du palladium, de l'argent, de l'hafnium, du tungstène, de l'iridium, un muscle artificiel, un alliage à mémoire de forme, ou une combinaison de ceux-ci.

5. Dispositif d'éversion de tissu selon la revendication 1, dans lequel ledit élément pénétrant le tissu est mobile par rapport à ladite gaine de distribution le long d'un axe de ladite gaine de distribution et dans lequel un mouvement dudit élément pénétrant le tissu le long de l'axe au-delà de l'extrémité de ladite gaine de distribution fait déplacer ledit élément pénétrant le tissu vers la deuxième configuration, et dans lequel un mouvement dudit élément pénétrant le tissu à l'extrémité de ladite gaine de distribution fait déplacer ledit élément pénétrant le tissu vers la première configuration.

6. Dispositif d'éversion de tissu selon la revendication 1, dans lequel ledit élément pénétrant le tissu peut être actionné pour atteindre la deuxième configuration par l'action d'un piston et de brides montés avec le dispositif.

7. Dispositif d'éversion de tissu selon la revendication 1, comprenant en outre une pluralité de lumières individuelles à l'intérieur de ladite gaine de distribution, et dans lequel lesdits éléments allongés sont montés de manière mobile à l'intérieur desdites lumières individuelles.
